# EUROPEAN PATENT APPLICATION

(11) **EP 1 837 034 A1**
(43) Date of publication of application: **26.09.2007**
(21) Application number: 05814590.5
(22) Date of filing: 30.11.2005
(51) Int. Cl.: A61K 45/00, A61K 31/7088, A61K 38/22, A61K 38/43, A61K 38/27, A61K 48/00, A61P 25/28, A61P 43/00

(54) **REMEDY FOR ALZHEIMER´S DISEASE**

(30) Priority: 30.11.2004 JP 2004347861
(71) Applicant: AnGes MG, Inc., Ibaraki-shi, Osaka 5670085 (JP); OSAKA UNIVERSITY, Suita-shi, Osaka 565-0871 (JP)
(72) Inventor: HAYASHI, Shinichiro, OSAKA UNIVERSITY, Suita-shi, Osaka 565-0871 (JP); SATO, Naoyuki, OSAKA UNIVERSITY, Suita-shi, Osaka 565-0871 (JP); TAKEUCHI, Daisuke, OSAKA UNIVERSITY, Suita-shi, Osaka 565-0871 (JP); MORISHITA, Ryuichi, OSAKA UNIVERSITY, Suita-shi, Osaka 565-0871 (JP)
(74) Representative: Gille Hrabal Struck Neidlein Prop Roos
(86) International application number: PCT/JP2005/022406
(87) International publication number: WO 2006/059777

(57) **Abstract**

The present invention provides a prophylactic/therapeutic agent for Alzheimer's disease comprising a substance that inhibits the action of β-amyloid 1-40 to suppress vascular endothelial precursor cell differentiation and/or endothelial precursor differentiation from stem cells and/or a substance that promotes vascularization as an active ingredient. The present invention also provides a screening method for a substance that inhibits the action of β-amyloid 1-40 to suppress vascular endothelial precursor cell differentiation and/or endothelial precursor differentiation from stem cells and/or a substance that promotes vascularization.

## Description

### TECHNICAL FIELD OF THE INVENTION

The present invention relates to a prophylactic/therapeutic agent for Alzheimer's disease comprising a substance that inhibits the action of β-amyloid 1-40 (hereinafter also referred to as Aβ1-40) to suppress vascular endothelial precursor cell differentiation and/or endothelial precursor differentiation from stem cells and/or a substance that promotes vascularization as an active ingredient. The present invention also relates to a prophylactic/therapeutic agent for Alzheimer's disease comprising a substance that inhibits the action of Aβ1-40 to suppress protein kinase B (hereinafter also referred to as Akt/PKB) activity as an active ingredient. Furthermore, the present invention still also relates to a screening method for a substance that inhibits the action of Aβ1-40 to suppress vascular endothelial precursor cell differentiation, a substance that inhibits the action of Aβ1-40 to suppress endothelial precursor differentiation from stem cells, and a substance that inhibits the action of Aβ1-40 to suppress Akt/PKB activity, which can become prophylactic/therapeutic agents for Alzheimer's disease.

### BACKGROUND OF THE INVENTION

Alzheimer's disease is a degenerative disease of the brain that represents senile dementia, and the number of patients is increasing year by year. In patients with Alzheimer's disease, reduction in their quality of life as a result of brain nervous dysfunction has a major impact not only on the patients, but also on surrounding people, including their families, and poses a serious problem in today's aging society. Although research into Alzheimer's disease is ongoing with great effort, the entire onset mechanism still remains unclear, and no radical drug has been developed.

Alzheimer's disease is described as a diverse syndrome; a large number of pathological findings have made it known that neurodegeneration and β-amyloid (hereinafter also referred to as Aβ) production/accumulation in the brain are important factors. Aβ is the primary component of senile plaques, which represent one of the primary neuropathological changes in Alzheimer's disease, and is produced upon cleavage of amyloid precursor protein (APP) by two kinds (β- and γ-) of secretase. There are two principal molecular species thereof: Aβ1-40, which consists of 40 amino acid residues, and Aβ1-42, which is longer by two residues on the C-terminal side. Aβ exhibits cytotoxicity to nerve cells; this action is more conspicuous in the state wherein Aβ aggregates and further fibrillates than in the single-molecule state. The aggregation rate of Aβ varies depending on the length of C-terminal portion; the aggregation and fibrillation are more likely in Aβ1-42 than in Aβ1-40. Therefore, despite the fact that about 90% of the extracellularly secreted Aβ is Aβ1-40, the principal molecular species of Aβ that accumulates in cerebral parenchyma is Aβ1-42. It is also known that all pathogenic mutations found in presenilin (PS), which is responsible for γ-secretase activity, increase Aβ1-42 production relative to Aβ1-40 production. Therefore, it is doubtless that Aβ1-42 is very important in the onset and progression of Alzheimer's disease.

Aβ1-42 is localized mainly in senile plaques and little occurs in cerebral blood vessels, whereas Aβ1-40 is localized mainly in cerebral blood vessels, rather than in senile plaques (see Stephen A. et al., J. Biol. Chem., 270(13): 7013-16, 1995). On the basis of the finding that if Aβ1-40 production becomes predominant over Aβ1-42 production, deposition of Aβ1-40 on vascular wall cells is promoted and cerebral amyloid angiopathy (CAA) is caused, a method of treating CAA using an Aβ1-40 inhibitor has been described (see Japanese Patent Kohyo Publication No. 2003-532656). However, no definite evidence has been found to date that Aβ1-40 triggers the onset of Alzheimer's disease.

Resent research has shown that neurogenesis and angiogenesis are closely associated with each other. However, traditional research into Alzheimer's disease has focused on neurodegeneration such as Aβ production/accumulation and neurofibrillation (due to accumulation of abnormally phosphorylated tau protein). For example, as prophylactic and therapeutic measures for Alzheimer's disease, a vaccine therapy based on immune responses to Aβ (see Japanese Patent Kohyo Publication No. 2002-502802), a method of inhibiting β-secretase or γ-secretase to prevent Aβ production, and the like have been reported.

In recent years, aspects of vascular disorders in Alzheimer's disease have come to draw attention, as seen in reports such as that progressive blood flow reduction and metabolic reduction due to cerebral atherosclerosis cause the earliest stage of Alzheimer's disease (see Rafael H. et al., Stroke 34 (8):e106. Epub, 2003), and that cerebral ischemia damages the blood brain barrier and upregulates intracerebral APP and ApoE to accelerate Aβ deposition (see Sadowski, Pankiewicz et al., Neurochemical Research, vol.29, No.6, 2004). However, much remains unclear how vascular disorders are associated with the onset and progression of Alzheimer's disease.

Accordingly, it is a problem to be solved by the present invention to elucidate the relationship between the onset/progression of Alzheimer's disease and vascular disorders, and to provide a novel prophylactic/therapeutic agent for Alzheimer's disease based on the relationship. Another problem to be solved by the present invention is to provide a method of screening for a prophylactic/therapeutic substance for Alzheimer's disease based on the above-described novel action mechanism.

### Disclosure of the Invention

The present inventors conducted diligent investigations to solve the above-described problems, and found that Aβ1-40, which is known to be localized in blood vessels, inhibited the differentiation, growth, migration, and vessel formation of vascular endothelial cells, suppression of endothelial precursor differentiation from stem cells, and vascular genesis/maturation. Furthermore, the present inventors found that the above-described actions of Aβ1-40 were based on inhibition of the Akt/PKB pathway activated by VEGF stimulation.

The present inventors conducted further investigations based on these findings, and developed the present invention.

Accordingly, the present invention provides:
(1) a prophylactic/therapeutic agent for Alzheimer's disease comprising a substance that inhibits the action of β-amyloid 1-40 to suppress vascular endothelial precursor cell differentiation and/or endothelial precursor differentiation from stem cells and/or a substance that promotes vascularization as an active ingredient,
(2) the agent described in (1), wherein the substance that inhibits the action of β-amyloid 1-40 to suppress vascular endothelial precursor cell differentiation and/or endothelial precursor differentiation from stem cells and/or the substance that promotes vascularization is a cytokine,
(3) the agent described in (2), wherein the cytokine that inhibits the action of β-amyloid 1-40 to suppress vascular endothelial precursor cell differentiation and/or endothelial precursor differentiation from stem cells is one or more selected from the group consisting of hepatocyte growth factor (HGF), vascular endothelial growth factor (VEGF), fibroblast growth factor (FGF), epidermal growth factor (EGF), insulin-like growth factor (IGF) and nerve growth factor (NGF),
(4) the agent described in (2), wherein the cytokine that promotes vascularization is one or more selected from the group consisting of hepatocyte growth factor (HGF), vascular endothelial growth factor (VEGF), fibroblast growth factor (FGF), epidermal growth factor (EGF), platelet-derived growth factor (PDGF), thymidine phosphorylase (TdRPase), platelet-derived endothelial cell growth factor (PD-ECGF), insulin-like growth factor (IGF) and nerve growth factor (NGF),
(5) a prophylactic/therapeutic agent for Alzheimer's disease comprising a nucleic acid that encodes a protein that inhibits the action of β-amyloid 1-40 to suppress vascular endothelial precursor cell differentiation and/or endothelial precursor differentiation from stem cells and/or a protein that promotes vascularization,
(6) a screening method for a substance that inhibits the action of β-amyloid 1-40 to suppress vascular endothelial precursor cell differentiation, which comprises bringing β-amyloid 1-40 into contact with vascular endothelial precursor cells in the presence and absence of a test substance, and comparing the degree of differentiation of the cells under the two conditions,
(7) a screening method for a substance that inhibits the action of β-amyloid 1-40 to suppress endothelial precursor differentiation from stem cells, which comprises bringing β-amyloid 1-40 into contact with stem cells in the presence and absence of a test substance, and comparing the degree of differentiation of the cells into the endothelial precursor under the two conditions,
(8) the method described in (6) or (7), which is a screening method for a prophylactic/therapeutic substance for Alzheimer's disease,
(9) a screening method for a prophylactic/therapeutic substance for Alzheimer's disease, which comprises culturing cells in which vascularization can be induced in the presence and absence of a test substance, and comparing the degree of vascularization in the cells under the two conditions,
(10) a prophylactic/therapeutic agent for Alzheimer's disease comprising a substance that inhibits the action of β-amyloid 1-40 to suppress protein kinase B activity and/or a substance that promotes protein kinase B activity as an active ingredient,
(11) the agent described in (10), wherein the substance that inhibits the action of β-amyloid 1-40 to suppress protein kinase B activity and/or the substance that promotes protein kinase B activity is a cytokine,
(12) the agent described in (11), wherein the cytokine is one or more selected from the group consisting of hepatocyte growth factor (HGF), vascular endothelial growth factor (VEGF), fibroblast growth factor (FGF), epidermal growth factor (EGF), insulin-like growth factor (IGF), nerve growth factor (NGF), platelet-derived growth factor (PDGF), thymidine phosphorylase (TdRPase), platelet-derived endothelial cell growth factor (PD-ECGF), insulin-like growth factor (IGF) and nerve growth factor (NGF),
(13) a prophylactic/therapeutic agent for Alzheimer's disease comprising a nucleic acid that encodes a protein that inhibits the action of β-amyloid 1-40 to suppress protein kinase B activity and/or a substance that promotes protein kinase B activity,
(14) a screening method for a substance that inhibits the action of β-amyloid 1-40 to suppress protein kinase B activity, which comprises bringing β-amyloid 1-40 into contact with cells capable of expressing protein kinase B activity in the presence and absence of a test substance, and comparing protein kinase B activity under the two conditions,
(15) the method described in (14), which is a screening method for a prophylactic/therapeutic substance for Alzheimer's disease,
(16) a screening method for a prophylactic/therapeutic substance for Alzheimer's disease, which comprises culturing cells capable of expressing protein kinase B activity in the presence and absence of a test substance, and comparing protein kinase B activity in the cells under the two conditions,
(17) a prophylactic/therapeutic method for Alzheimer's disease, which comprises administering a substance that inhibits the action of β-amyloid 1-40 to suppress vascular endothelial precursor cell differentiation and/or endothelial precursor differentiation from stem cells and/or a substance that promotes vascularization,
(18) a prophylactic/therapeutic method for Alzheimer's disease, which comprises administering a substance that inhibits the action of β-amyloid 1-40 to suppress protein kinase B activity and/or a substance that promotes protein kinase B activity,
(19) a use of a substance that inhibits the action of β-amyloid 1-40 to suppress vascular endothelial precursor cell differentiation and/or endothelial precursor differentiation from stem cells and/or a substance that promotes vascularization, for manufacturing a prophylactic/therapeutic agent for Alzheimer's disease, and
(20) a use of a substance that inhibits the action of β-amyloid 1-40 to suppress protein kinase B activity and/or a substance that promotes protein kinase B activity, for manufacturing a prophylactic/therapeutic agent for Alzheimer's disease.

### Brief Description of the Drawings

Figure 1 is a graph showing the results of Example 1.
Figure 2 presents photomicrographs showing the results of Example 1.
Figure 3 is a graph showing the results of Example 2.
Figure 4 presents photomicrographs showing the results of Example 2.
Figure 5 is a graph showing the results of Example 3.
Figure 6 is a graph showing the results of Example 4 (blood vessel density).
Figure 7 is a graph showing the results of Example 4 (hemoglobin content).
Figure 8 is a graph showing the results of Example 5.
Figure 9 presents electrophoregrams showing the results of Example 6 (Akt).
Figure 10 is a graph showing the results of Example 6.
Figure 11 is an electrophoregram showing the results of Example 6 (Phosho-ERK, +VEGF).
Figure 12 is an electrophoregram showing the results of Example 6 (Phosho-ERK, +VEGF+Aβ1-40).
Figure 13 presents photomicrographs showing the results of Example 7.
Figure 14 is a graph showing the effect obtained when the human HGF gene was introduced in Test Example 1 (water finding test).
Figure 15 is a graph showing the effect obtained when the human HGF gene was introduced in Test Example 2 (Y-Maze test).

### Detailed Description of the Invention

Modes of embodiment of the present invention are hereinafter described.

In the present invention, "a prophylactic/therapeutic agent" is used to prevent and treat conditions of an ill patient; treatment as mentioned herein refers to a concept that includes amelioration of symptoms and prevention of progression (exacerbation) of conditions or symptoms.

The prophylactic/therapeutic agent of the present invention is effective on mammals (for example, mice, rats, hamsters, rabbits, cats, dogs, cattle, sheep, monkeys, humans and the like), and is useful for Alzheimer's disease.

In the present invention, Alzheimer's disease is defined as a disease characterized by degeneration/deficit of brain nerve cells, and generically refers to the conditions caused thereby. Although the severity and symptoms are variable, all are covered by the prophylactic and therapeutic agents of the invention of this application.

The prophylactic/therapeutic agent of the present invention comprises as an active ingredient a substance that inhibits the action of Aβ1-40 to suppress vascular endothelial precursor cell differentiation and/or endothelial precursor differentiation from stem cells and/or a substance that promotes vascularization.

The substance that inhibits the action of Aβ1-40 to suppress vascular endothelial precursor cell differentiation and/or endothelial precursor differentiation from stem cells and/or the substance that promotes vascularization is not subject to limitation, as long as it exhibits the action; for example, cytokines and the like can be mentioned. The term cytokine as used herein is intended to represent the meaning and concept in common use in the art.

As examples of the cytokine that inhibits the action of Aβ1-40 to suppress vascular endothelial precursor cell differentiation and/or endothelial precursor differentiation from stem cells, hepatocyte growth factor (HGF), which is produced by various mesenchymal cells; vascular endothelial growth factor (VEGF), which is produced in cells such as macrophages, smooth muscle cells, and tumor cells; fibroblast growth factor (FGF), which is secreted from various cells, including endothelial cells; epidermal growth factor (EGF), which is known to be relatively abundantly present in saliva and urine; insulin-like growth factor (IGF), which promotes the growth of a wide variety of cells; nerve growth factor (NGF), which is a kind of neurotrophic factor; and the like can be mentioned, preferably HGF, VEGF, FGF and EGF, more preferably HGF, VEGF and FGF.

As examples of the cytokine that promotes vascularization, thymidine phosphorylase (TdRPase), an enzyme involved in pyrimidine nucleoside metabolism, and platelet-derived endothelial cell growth factor (PD-ECGF), which is purified from platelets, and the like, in addition to hepatocyte growth factor (HGF), vascular endothelial growth factor (VEGF), fibroblast growth factor (FGF), epidermal growth factor (EGF), platelet-derived growth factor (PDGF), which is present in serum, insulin-like growth factor (IGF), and nerve growth factor (NGF), can be mentioned, preferably HGF, VEGF, FGF and EGF.

The cytokine of the present invention may be a protein derived from a cell (e.g., hepatocyte, splenocyte, nerve cell, glial cell, pancreatic β cell, myeloid cell, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding precursor cell, stem cell or cancer cell thereof, and the like) of a warm-blooded animal (for example, human, mouse, rat, guinea pig, hamster, rabbit, sheep, goat, swine, bovine, horse, bird, cat, dog, monkey, chimpanzee and the like), or any tissue where such cells are present (e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue, skeletal muscle, and the like) and may also be a chemically synthesized protein or a protein synthesized using a cell-free translation system. Alternatively, the cytokine may be a recombinant protein produced by a transformant introduced with a nucleic acid having the base sequence that encodes the above-described protein.

The cytokine may have any of a carboxyl group (-COOH), a carboxylate (-COO⁻), an amide (-CONH₂) or an ester (-COOR) at the C-terminus thereof.

As used herein, R in the ester is exemplified by C₁₋₆ alkyl groups, for example, methyl, ethyl, n-propyl, isopropyl, n-butyl and the like; C₃₋₈ cycloalkyl groups, for example, cyclopentyl, cyclohexyl and the like; C₆₋₁₂ aryl groups, for example, phenyl, α-naphthyl and the like; phenyl-C₁₋₂ alkyl groups, for example, benzyl, phenethyl and the like; C₇₋₁₄ aralkyl groups such as α-naphthyl-C₁₋₂-alkyl groups such as α-naphthylmethyl; pivaloyloxymethyl groups; and the like.

When the cytokine has a carboxyl group (or carboxylate) at a position other than the C-terminus, the carboxyl group may be amidated or esterified, and such proteins are also included in the cytokine of the present invention. As examples of the ester used in this case, the above-described esters at the C-terminus can be mentioned.

Furthermore, the cytokine also includes those wherein the amino group of the amino acid residue at the N-terminus (e.g., methionine residue) is protected with a protecting group (for example, C₁₋₆ acyl groups such as C₁₋₆ alkanoyls such as formyl group and acetyl group); those wherein the glutamine residue at the N-terminus, which is produced upon cleavage in vivo, is pyroglutaminated; those wherein a substituent (for example, -OH, -SH, amino group, imidazole group, indole group, guanidino group and the like) on the side chain of an amino acid in the molecule is protected with an appropriate protecting group (for example, C₁₋₆ acyl groups such as C₁₋₆ alkanoyl groups such as formyl group and acetyl group, and the like); conjugated proteins such as what are called glycoproteins having sugar chains bound thereto, and the like.

The cytokine can be a physiologically acceptable salt with an acid or a base, preferably a physiologically acceptable acid addition salt. Useful salts include, but not limited to, for example, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

When isolation and purification of cytokine from the aforementioned cells and tissues and the like are to be conducted, after homogenizing the cells and tissues according to a conventional method, protein isolation methods know per se, such as methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing can be combined as appropriate. When the cytokine thus obtained is in a free form, the free form can be converted to a salt by a method known per se or a method based thereon; when the protein is obtained as a salt, the salt can be converted to a free form or other salt by a method known per se or a method based thereon.

The cytokine used in the present invention may be a full-length protein or a fragment (partial peptide) thereof, as long as it inhibits the action of Aβ1-40 to suppress vascular endothelial precursor cell differentiation and/or endothelial precursor differentiation from stem cells and/or promotes vascularization.

The cytokine used in the present invention or a partial peptide thereof can also be produced according to a commonly known method of peptide synthesis.

The peptide synthesis process may be any of, for example, a solid phase synthesis process and a liquid phase synthesis process. A desired protein can be produced by condensing a partial peptide or amino acids capable of constituting cytokine with the remaining portion, and removing the protecting group if any in the resultant product.

Here, the condensation and the removal of the protecting group are conducted according to methods known per se, for example, methods described in [1] to [5] below.
[1] M. Bodanszky and M.A. Ondetti: Peptide Synthesis, Interscience Publishers, New York (1966)
[2] Schroeder and Luebke: The Peptide, Academic Press, New York (1965)
[3] Nobuo Izumiya, et al.: Peptide Gosei-no-Kiso to Jikken, published by Maruzen Co. (1975);
[4] Haruaki Yajima and Shunpei Sakakibara: Seikagaku Jikken Koza 1, Tanpakushitsu no Kagaku IV, 205 (1977)
[5] Haruaki Yajima, ed.: Zoku Iyakuhin no Kaihatsu, Vol. 14, Peptide Synthesis, published by Hirokawa Shoten.

The cytokine or a partial peptide thereof thus obtained can be isolated and purified by a publicly known method of purification. Here, as examples of the method of purification, solvent extraction, distillation, column chromatography, liquid chromatography, recrystallization, a combination thereof, and the like can be mentioned.

When the cytokine or a partial peptide thereof obtained by the above-described method is a free form, the free form can be converted to an appropriate salt by a publicly known method or a method based thereon; conversely, when the cytokine or a partial peptide thereof is obtained in the form of a salt, the salt can be converted to a free form or another salt by a publicly known method or a method based thereon.

The partial peptide of the cytokine or a salt thereof can also be produced by cleaving the full length protein or a salt thereof with an appropriate peptidase.

Furthermore, the cytokine of the present invention or a partial peptide thereof can also be produced by culturing a transformant comprising a nucleic acid encoding the cytokine or a partial peptide thereof, and separating and purifying the cytokine or a partial peptide thereof from the culture obtained.

The nucleic acid encoding the cytokine or a partial peptide thereof may be a DNA or RNA, or may be a DNA/RNA chimera. The nucleic acid is preferably a DNA. Also, the nucleic acid may be double stranded or single stranded. When the nucleic acid is double stranded, it may be a double-stranded DNA, a double-stranded RNA or a DNA:RNA hybrid. When the nucleic acid is single stranded, it may be a sense strand (i.e., coding strand) or an antisense strand (i.e., non-coding strand).

As the DNA encoding cytokine or a partial peptide thereof, genomic DNA, cDNA derived from any cell [for example, splenocyte, nerve cell, glial cell, pancreatic β cells, myeloid cell, mesangial cell, Langerhans' cell, epidermal cell, epithelial cell, endothelial cell, fibroblast, fibrocyte, myocytes, adipocyte, immune cell (e.g., macrophage, T cell, B cell, natural killer cell, mast cell, neutrophil, basophil, eosinophils, monocyte), megakaryocyte, synovial cell, chondrocytes, bone cell, osteoblast, osteoclast, mammary cell, hepatocyte or interstitial cell, or a corresponding precursor cell, stem cell, cancer cell thereof and the like, blood cells] of warm-blooded animal (e.g., human, mouse, rat, guinea pig, hamster, rabbit, sheep, goat, swine, bovine, horse, bird, cat, dog, monkey, chimpanzee and the like), or any tissue where such cells are present [e.g., brain or any portion of brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, lateral lobe, putamen, caudate nucleus, callosum, substantia nigra), spinal cord, hypophysis, stomach, pancreas, kidney, liver, gonad, thyroid, gallbladder, bone marrow, adrenal gland, skin, muscle, lung, gastrointestinal tract(e.g., large intestine, small intestine), blood vessel, heart, thymus, spleen, submandibular gland, peripheral blood, peripheral blood cell, prostate, testicle, ovary, placenta, uterus, bone, joint, skeletal muscle, and the like], synthetic DNA and the like can be mentioned. A genomic DNA and cDNA encoding cytokine or a partial peptide thereof can also be directly amplified by Polymerase Chain Reaction (hereinafter to be abbreviated as "PCR method") or Reverse Transcriptase-PCR (hereinafter to be abbreviated as "RT-PCR method"), using genomic DNA fraction or whole RNA or mRNA fraction prepared from the above-mentioned cell·tissue as a template. Alternatively, genomic DNA or cDNA encoding cytokine or a partial peptide thereof can also be cloned by colony or plaque hybridization method, PCR method and the like, from the genomic DNA library or cDNA library prepared by inserting, into a suitable vector, a fragment of genomic DNA and whole RNA or mRNA prepared from the above-mentioned cell· tissue. The vector to be used for the library may be any of bacteriophage, plasmid, cosmid, phagemid and the like.

The DNA that encodes the cytokine or a partial peptide thereof can be cloned by amplifying it by the PCR method using a synthetic DNA primer having a portion of the base sequence that encodes the protein or peptide, or by hybridizing DNA incorporated in an appropriate expression vector to a labeled DNA fragment or synthetic DNA that encodes a portion or the entire region of the protein of the present invention. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known *per se,* such as the ODA-LA PCR method, the Gapped duplex method, the Kunkel method and the like, or a method based thereon, using a publicly known kit, for example, Mutan^{™}-super Express Km (Takara Shuzo Co., Ltd.), Mutan^{™}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

The protein or peptide can be produced by transforming a host with an expression vector containing a DNA encoding the above-mentioned cytokine or a partial peptide thereof and cultivating the obtained transformant.

An expression vector containing a DNA encoding cytokine or a partial peptide thereof can be produced, for example, by cleaving out an object DNA fragment from the DNA encoding cytokine and connecting the DNA fragment downstream of a promoter in a suitable expression vector.

Useful expression vectors include plasmids derived from *E. coli* (e.g., pBR322, pBR325, pUC12, pUC13); plasmids derived from *Bacillus subtilis* (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo and the like.

The promoter may be any promoter, as long as it is appropriate for the host used to express the gene.

For example, when the host is an animal cell, the SRα promoter, the SV40 promoter, the LTR promoter, the CMV (cytomegalovirus) promoter, the HSV-TK promoter and the like are used. Of these, the CMV promoter, the SRα promoter and the like are preferred.

When the host is a bacterium of the genus *Escherichia,* the trp promoter, the lac promoter, the recA promoter, the λP_{L} promoter, the lpp promoter, the T7 promoter and the like are preferred.

When the host is a bacterium of the genus *Bacillus,* the SPO1 promoter, the SPO2 promoter, the penP promoter and the like are preferred.

When the host is yeast, the PHO5 promoter, the PGK promoter, the GAP promoter, the ADH promoter and the like are preferred.

When the host is an insect cell, the polyhedrin prompter, the P10 promoter and the like are preferred.

Useful expression vectors include, in addition to the above, expression vectors that optionally comprises an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene defective Chinese hamster cell is used and the dhfr gene is used as the selection marker, a target gene can also be selected using a thymidine-free medium.

Where necessary, a base sequence encoding (signal codon) a signal sequence suitable for the host may be added to the 5' end side of DNA encoding cytokine or a partial peptide thereof. When the host is a bacterium of the genus *Escherichia,* PhoA· signal sequence, OmpA·signal sequence and the like are used; when the host is a bacterium of the genus *Bacillus*, α-amylase· signal sequence, subtilisin·signal sequence and the like are used; when the host is yeast, MFα·signal sequence, SUC2·signal sequence and the like are used; and when the host is an animal cell, insulin·signal sequence, α-interferon·signal sequence, antibody molecule ·signal sequence and the like are used.

As useful examples of the host, a bacterium of the genus *Escherichia,* a bacterium of the genus *Bacillus,* yeast, an insect cell, an insect, an animal cell, and the like can be mentioned.

As useful examples of the bacterium of the genus *Escherichia, Escherichia coli* K12 DH1 *(*Proc. Natl. Acad. Sci. U.S.A., Vol. 60, 160 (1968)), JM103 *(*Nucleic Acids Research, Vol. 9, 309 (1981)), JA221 *(*Journal of Molecular Biology, Vol. 120, 517 (1978)), HB101 *(*Journal of Molecular Biology, Vol. 41, 459 (1969)), C600 *(*Genetics, Vol. 39, 440 (1954)), and the like can be mentioned.

As useful examples of the bacterium of the genus *Bacillus, Bacillus subtilis* MI114 *(*Gene, Vol. 24, 255 (1983)), 207-21 *(*Journal of Biochemistry, Vol. 95, 87 (1984)) and the like can be mentioned.

As useful examples of the yeast, *Saccharomyces cerevisiae* AH22, AH22R⁻, NA87-11A, DKD-5D and 20B-12, *Schizosaccharomyces pombe* NCYC1913 and NCYC2036, *Pichia pastoris* KM71 and the like can be mentioned.

As useful examples of the insect cell, *Spodoptera frugiperda* cell (Sf cell), MG1 cell derived from the mid-intestine of *Trichoplusia ni,* High Five^{™} cell derived from an egg of *Trichoplusia ni,* cell derived from *Mamestra brassicae,* cell derived from *Estigmena acrea,* and the like can be mentioned when the virus is AcNPV. When the virus is BmNPV, useful insect cells include *Bombyx mori* N cell (BmN cell) and the like. As useful examples of the Sf cell, Sf9 cell (ATCC CRL1711), Sf21 cell (both in Vaughn, J. L. et al., In Vivo, 13, 213-217 (1977)), and the like can be mentioned.

As useful examples of the insect, a larva of Bombyx mori (Maeda et al., Nature, 315, 592 (1985)), and the like can be mentioned.

As useful examples of the animal cell, monkey cell COS-7, Vero, Chinese hamster cell CHO (hereafter abbreviated as CHO cell), dhfr gene defective Chinese hamster cell CHO (hereafter abbreviated as CHO(dhfr⁻) cell), mouse L cell, mouse AtT-20, mouse myeloma cell, rat GH3, human FL cell and the like can be mentioned.

Transformation can be carried out according to the kind of host in accordance with a publicly known method.

A bacterium of the genus *Escherichia* can be transformed, for example, in accordance with a method described in Proc. Natl. Acad. Sci. U.S.A., 69, 2110 (1972), Gene, 17, 107 (1982), and the like.

A bacterium of the genus *Bacillus* can be transformed, for example, according to a method described in Molecular and General Genetics, 168, 111 (1979), and the like.

Yeast can be transformed, for example, in accordance with a method described in Methods in Enzymology, 194, 182-187 (1991), Proc. Natl. Acad. Sci. USA, 75, 1929 (1978), and the like.

An insect cell and an insect can be transformed, for example, according to a method described in Bio/Technology, 6, 47-55 (1988), and the like.

An animal cell can be transformed, for example, in accordance with a method described in Saibo Kogaku, extra issue 8, Shin Saibo Kogaku Jikken Protocol, 263-267 (1995), published by Shujunsha, or Virology, 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably comprises a carbon source, a nitrogen source, an inorganic substance, and the like, necessary for the growth of the transformant. Here, as examples of the carbon source, glucose, dextrin, soluble starch, sucrose, and the like can be mentioned; as examples of the nitrogen source, inorganic and organic substances such as an ammonium salt, a nitrate salt, corn steep liquor, peptone, casein, meat extract, soybean cake, potato extract, and the like can be mentioned; as examples of the inorganic substance, calcium chloride, sodium dihydrogen phosphate, magnesium chloride, and the like can be mentioned. In addition, the medium may be supplemented with yeast extract, vitamins, growth promoting factor, and the like. Preferably, the pH of the medium is about 5 to 8.

Preferable examples of the medium used to cultivate a transformant whose host is a bacterium of the genus *Escherichia* include a M9 medium supplemented with glucose and a Casamino acid (Miller, Journal of Experiments in Molecular Genetics, 431-433, Cold Spring Harbor Laboratory, New York, 1972). As required, in order to increase promoter efficiency, a chemical such as 3β-indolylacrylic acid may be added to the medium.

Cultivation of a transformant whose host is a bacterium of the genus *Escherichia* is normally carried out at about 15°C to 43°C for about 3 to 24 hours. As necessary, the culture may be aerated or agitated.

Cultivation of a transformant whose host is a bacterium of the genus *Bacillus* is normally carried out at about 30°C to 40°C for about 6 to 24 hours. As necessary, the culture may be aerated or agitated.

As examples of the medium for cultivating a transformant whose host is yeast, Burkholder's minimum medium [Bostian, K.L. et al., Proc. Natl. Acad. Sci. USA, 77, 4505 (1980)] and SD medium supplemented with 0.5% casamino acid [Bitter, G.A. et al., Proc. Natl. Acad. Sci. USA, 81, 5330 (1984)] can be mentioned. The medium's pH is preferably about 5 to 8. Cultivation is normally carried out at about 20°C to 35°C for about 24 to 72 hours. As necessary, the culture may be aerated or agitated.

Useful medium for cultivating a transformant whose host is an insect cell or an insect include, for example, Grace's Insect Medium [Grace, T.C.C., Nature, 195, 788 (1962)] supplemented with additives such as 10% inactivated bovine serum as appropriate. The medium's pH is preferably about 6.2 to 6.4. Cultivation is normally carried out at about 27°C for about 3 to 5 days. As necessary, the culture may be aerated or agitated.

Useful medium for cultivating a transformant whose host is an animal cell include, for example, MEM medium *[*Science, 122, 501(1952)], DMEM medium *[Virology,* 8, 396(1959)], RPMI 1640 medium *[*The Journal of the American Medical Association, 199, 519(1967)], 199 medium *[*Proceeding of the Society for the Biological Medicine, 73, 1(1950)] and the like, supplemented with about 5 to 20% fetal bovine serum. The medium's pH is preferably about 6 to 8. Cultivation is normally carried out at about 30°C to 40°C for about 15 to 60 hours. As necessary, the culture may be aerated or agitated.

As described above, the cytokine or a partial peptide thereof can be produced in or outside the cells of the transformant.

The cytokine or a partial peptide thereof can be separated and purified from the culture obtained by cultivating the aforementioned transformant, according to a method known *per se.*

For example, when the cytokine or a partial peptide thereof is extracted from cultivated bacteria or cells, a method is used as appropriate wherein the bacteria or cells are recovered from the culture by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100^{™}.

Isolation and purification of the cytokine or a partial peptide thereof contained in the thus-obtained soluble fraction can be conducted according to a method know *per se.* Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing, and the like. These methods can be combined as appropriate.

When the thus-obtained cytokine or a partial peptide thereof is a free form, the free form can be converted to a salt by a method known *per se* or a method based thereon; when the protein or the peptide is obtained as a salt, the salt can be converted to a free form or another salt by a method known *per* se or a method based thereon.

Note that the cytokine or a partial peptide thereof produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained cytokine or a partial peptide thereof can be confirmed by enzyme immunoassay, Western blotting and the like employing an antibody having a specific affinity thereto.

Furthermore, the cytokine or a partial peptide thereof can also be synthesized by in vitro translation using a cell-free protein translation system that comprises a rabbit reticulocyte lysate, wheat germ lysate, *Escherichia coli* lysate and the like, with RNA corresponding to DNA that encodes cytokine or a partial peptide thereof as the template. Alternatively, the cytokine or a partial peptide thereof can be synthesized using a cell-free transcription/translation system further containing RNA polymerase, with the DNA that encodes the cytokine or a partial peptide thereof as the template. As the cell-free protein (transcription/) translation system, commercially available ones can be used, an *Escherichia coli* extract can also be prepared by a method known *per se,* and specifically, the method described in Pratt J. M. et al., Transcription and Translation, 179-209, Hames B. D. & Higgins S. J. eds., IRL Press, Oxford (1984) and the like. As the commercially available cell lysates, as those derived from *Escherichia coli, E. coli* S30 extract system (manufactured by Promega), RTS 500 Rapid Translation System (manufactured by Roche) and the like can be mentioned, as those derived from rabbit reticulocyte, Rabbit Reticulocyte Lysate System (manufactured by Promega) and the like can be mentioned, and as those derived from wheat germ, PROTEIOS^{™} (manufactured by TOYOBO) and the like can be mentioned. Of these, ones using a wheat germ lysate are preferable. As the production method of wheat germ lysate, for example, the methods described in Johnston F.B. et al., Nature, 179: 160-161 (1957) or Erickson A.H. et al., Meth. Enzymol., 96: 38-50 (1996) and the like can be used.

As the system or apparatus for protein synthesis, batch method (Pratt, J.M. et al. (1984), mentioned above), continuous cell-free protein synthesis system (Spirin A.S. et al., Science, 242: 1162-1164 (1988)) wherein amino acid, energy source and the like are continuously supplied to the reaction system, dialysis (Kikawa et al., The 21st Annual Meeting of the Molecular Biology Society of Japan, WID6), or overlay method (manual of PROTEIOS^{™} Wheat germ cell-free protein synthesis core kit: manufactured by TOYOBO) and the like can be mentioned. Moreover, a method (JP-A-2000-333673) wherein template RNA, amino acid, energy source and the like are supplied to a synthesis reaction system as necessary and a synthesized substance and decomposed product are discharged as necessary, and the like can be used.

Provided that the substance that inhibits the action of Aβ1-40 to suppress vascular endothelial precursor cell differentiation and/or endothelial precursor differentiation from stem cells and/or the substance that promotes vascularization is a protein or peptide such as the above-described cytokine or a partial peptide thereof, the substance can be formulated in the prophylactic/therapeutic agent of the present invention in the form of a nucleic acid that encodes the same, particularly an expression vector comprising the nucleic acid. As examples of the nucleic acid that encodes the cytokine or a partial peptide thereof, the above-described nucleic acid used to produce the cytokine or a partial peptide thereof can be mentioned. The expression vector must be one wherein the above-mentioned nucleic acid is functionally joined to a promoter capable of exhibiting promoter activity in the cells of the recipient mammal. Any promoter capable of functioning in the recipient mammal can be used; examples include viral promoters such as the SV40-derived early promoter, cytomegalovirus LTR, Rous sarcoma virus LTR, MoMuLV-derived LTR, and adenovirus-derived early promoter, and mammalian component protein gene promoters such as β-actin gene promoter, PGK gene promoter, and transferrin gene promoter and the like.

The expression vector preferably comprises a transcription termination signal, that is, a terminator region, downstream of the above-described nucleic acid. Furthermore, the expression vector may further comprise a selection marker gene for selecting transformant cells (genes that confer resistance to drugs such as tetracycline, ampicillin, kanamycin, hygromycin, and phosphinothricin, gene that compensate for auxotrophic mutation, and the like).

Although the basic backbone vector used as the expression vector is not subject to limitation, vectors suitable for administration to mammals such as humans include viral vectors such as adenovirus, retrovirus, adeno-associated virus, herpesvirus, vaccinia virus, poxvirus, poliovirus, Sindbis virus, and Sendai virus.

Administration of an expression vector comprising a nucleic acid that encodes a cytokine or a partial peptide thereof is conducted by either the *ex vivo* method, wherein cells of the animal subject of treatment are taken out from the body, cultured, and then transfected and returned to the body, or the in vivo method, wherein the vector is administered and introduced directly into the body of the recipient. In the case of the ex vivo method, vector introduction to the target cell can be performed by the microinjection method, calcium phosphate co-precipitation method, PEG method, electroporation method and the like. In the case of the in vivo method, the viral vector is administered intravenously, intra-arterially, subcutaneously, intracutaneously, intramuscularly, intraperitoneally and the like in the form of an injection and the like. Alternatively, when the vector is administered by intravenous injection and the like, production of a neutralizing antibody against the viral vector becomes problematic; however, provided that the vector is topically injected to the site where the target cell is present (*in situ* method), the adverse effect of the presence of the antibody can be mitigated.

Also, when a non-viral vector is used as the expression vector comprising a nucleic acid that encodes a cytokine or a partial peptide thereof, introduction of the expression vector can be performed using a polymeric carrier such as a polyL-lysine-nucleic acid complex or in a liposome-enveloped state. Alternatively, the vector can also be introduced directly to the target cell using the particle gun method.

The prophylactic/therapeutic agent of the present invention may comprise any carrier, for example, a pharmaceutically acceptable carrier, in addition to a substance that inhibits the action of Aβ1-40 to suppress vascular endothelial precursor cell differentiation and/or endothelial precursor differentiation from stem cells and/or a substance that promotes vascularization.

As examples of the pharmaceutically acceptable carrier, excipients such as sucrose, starch, mannitol, sorbitol, lactose, glucose, cellulose, talc, calcium phosphate and calcium carbonate; binders such as cellulose, methylcellulose, hydroxypropylcellulose, polypropylpyrrolidone, gelatin, acacia, polyethylene glycol, sucrose and starch; disintegrants such as starch, carboxymethylcellulose, hydroxypropyl starch, sodium-glycol-starch, sodium hydrogen carbonate, calcium phosphate and calcium citrate; lubricants such as magnesium stearate, Aerosil, talc and sodium lauryl sulfate; flavoring agents such as citric acid, menthol, glycyrrhizin ammonium salt, glycine and orange flour; preservatives such as sodium benzoate, sodium hydrogen sulfite, methyl paraben and propyl paraben; stabilizers such as citric acid, sodium citrate and acetic acid; suspending agents such as methylcellulose, polyvinylpyrrolidone and aluminum stearate; dispersing agents such as surfactants; diluents such as water, physiological saline and orange juice; base waxes such as cacao butter, polyethylene glycol and white beeswax; and the like can be mentioned, which, however, are not to be construed as limiting.

Preparations suitable for oral administration are a liquid comprising an effective amount of substance dissolved in a diluent like water, physiological saline or orange juice, a capsule, sachet or tablet containing an effective amount of substance as a solid or granules, a suspension comprising an effective amount of substance suspended in an appropriate dispersion medium, an emulsion comprising a solution of an effective amount of substance dispersed and emulsified in an appropriate dispersion medium, and the like.

As preparations suitable for parenteral administration (for example, intravenous injection, intramuscular injection, topical injection, intraperitoneal administration and the like), aqueous and non-aqueous isotonic sterile injectable liquids are available, which may contain an antioxidant, a buffer solution, a bacteriostatic agent, an isotonizing agent and the like. Also, aqueous and non-aqueous sterile suspensions can be mentioned, which may contain a suspending agent, a solubilizer, a thickener, a stabilizer, an antiseptic and the like. The preparation can be enclosed in a container for a unit dosage or a multiple dosage, like ampoules and vials. Also, an active ingredient and a pharmaceutically acceptable carrier can also be freeze-dried and stored in a state that may be dissolved or suspended in an appropriate sterile vehicle just before use.

Although the dosage of the prophylactic/therapeutic agent of the present invention varies depending on the activity and kind of active ingredient, seriousness of illness, recipient animal species, the recipient's drug tolerance, body weight, age, and the like, it is normally about 1 µg to about 1000 mg, preferably about 10 µg to about 500 mg, more preferably about 100 µg to about 100 mg, as the amount of active ingredient per day for an adult. This is administered at one time or in several divided portions.

The prophylactic/therapeutic agent of the present invention may be any one comprising one kind of either a substance that inhibits the action of Aβ1-40 to suppress vascular endothelial precursor cell differentiation and/or endothelial precursor differentiation from stem cells, or a substance that promotes vascularization, as an active ingredient, and may comprise both. It is also possible to use alone a substance having both actions. Furthermore, two or more kinds of one or both of a substance that inhibits the action of Aβ1-40 to suppress vascular endothelial precursor cell differentiation and a substance that promotes vascularization may be contained. Although the method of administration of the agent of the present invention may be an oral administration or a parenteral administration (ingestion) such as drip infusion or injection (transvenous administration) and is not subject to limitation, a topical administration such as muscular injection or subcutaneous administration is preferable when the active ingredient is a protein or a nucleic acid that encodes the same.

Substances that are active ingredients of the present invention may be prepared separately or in any combination, or may be prepared as a preparation containing all of them. If they are administered as separate preparations, the routes of administration and dosage forms thereof may be identical or not, and the timings of administration may be simultaneous or separate. These are determined as appropriate according to the kind and effect of drug for coadministration.

The present invention also provides a screening method for a substance that inhibits the action of Aβ1-40 to suppress vascular endothelial precursor cell differentiation, which comprises bringing Aβ1-40 into contact with vascular endothelial precursor cells in the presence and absence of a test substance, and comparing the degrees of differentiation of the cells under the two conditions, the substance obtainable by the screening method, and a prophylactic/therapeutic agent for Alzheimer's disease comprising the substance.

The test substance subjected to the screening method of the present invention may be any known compound and novel compound; for example, a nucleic acid, saccharide, lipid, protein, peptide, organic low molecular weight compound, a compound library prepared using combinatorial chemistry technology, a random peptide library prepared by solid phase synthesis or the phage display method, or naturally occurring ingredients derived from microorganisms, animals, plants, marine organisms and the like, and the like can be mentioned.

In one mode of embodiment, the screening method of the present invention comprises the following steps (I), (II) and (III):
(I) a step for bringing Aβ1-40 into contact with vascular endothelial precursor cells in the presence of a test substance,
(II) a step for measuring the degree of differentiation in the cells contacted in (I) above, and comparing it with the degree of differentiation in control cells brought into contact with Aβ1-40 in the absence of the test substance,
(III) a step for selecting a test substance that has inhibited the action of Aβ1-40 to suppress vascular endothelial precursor cell differentiation, on the basis of the results of the comparison in (II) above.

In step (I) of the above-described method, Aβ1-40 is brought into contact with vascular endothelial precursor cells in the presence of a test substance. The contact can be performed in culture medium. The cells can be animal-derived vascular endothelial precursor cells, for example, vascular endothelial precursor cells derived from mammals such as mice, rats, hamsters, guinea pigs, rabbits, dogs, monkeys, and humans. Regarding vascular endothelial cells, for example, human brain microvascular endothelial cells (HBMEC), human umbilical artery endothelial cells (HUAEC), human umbilical vein endothelial cells (HUVEC) and the like are commercially available, and can be purchased and utilized.

The culture medium wherein Aβ1-40 is brought into contact with vascular endothelial precursor cells is chosen as appropriate according to the kind of cells used and the like; for example, a minimal essential medium (MEM), Dulbecco's modified Eagle medium (DMEM), RPMI1640 medium, 199 medium and the like, comprising about 5 to 20% fetal bovine serum, can be mentioned. The medium may comprise an ingredient capable of inducing differentiation into endothelial cells; culturing conditions are also determined as appropriate according to the kind of cells used and the like; for example, the pH of the medium is about 6 to about 8, culturing temperature is normally about 30°C to about 40°C, and culturing time is about 12 hours to about for 72 hours. (See J. Clin. Invest., 1973 Nov; 52(11):2745-56)

In step (II) of the above-described method, first, the degree of suppression of cell differentiation in the cells contacted with Aβ1-40 is measured. The measurement can be performed by a method known per se in consideration of the kind of cells used and the like.

For example, an evaluation can be performed using a kit such as the BD Biocoat TM angiogenesis system, manufactured by Nippon Becton Dickinson Co., Ltd.
(http://www.bdj.co.jp/pdf/35-284-288.pdf)

Next, the degree of suppression of cell differentiation in the cells contacted in the presence of a test substance is compared with the degree of suppression of cell differentiation in the cells contacted in the absence of the test substance. The comparison of the degree of differentiation is preferably performed on the basis of the presence or absence of a significant difference. Although the degree of differentiation in the control cells contacted in the absence of the test substance may be a degree of differentiation measured prior to, or simultaneously with, the measurement of the degree of differentiation in the cells contacted in the presence of the test substance, it is preferable from the viewpoint of experimental accuracy and reproducibility that the degree of differentiation be a simultaneously measured degree of differentiation.

In step (III) of the above-described method, a test substance that has inhibited the action of Aβ1-40 to suppress vascular endothelial precursor cell differentiation, that is, a test substance that has promoted cell differentiation compared to the control cells is selected.

The present invention also provides a screening method for a substance that inhibits the action of Aβ1-40 to suppress endothelial precursor differentiation from stem cells, which comprises bringing Aβ1-40 into contact with stem cells in the presence and absence of a test substance, and comparing the degree of differentiation of the cells into the endothelial precursor under the two conditions, the substance obtainable by the screening method, and a prophylactic/therapeutic agent for Alzheimer's disease comprising the substance.

The test substance subjected to this screening method may be any commonly known compound or new compound; examples include nucleic acids, saccharides, lipids, proteins, peptides, organic low-molecular compounds, compound libraries prepared using combinatorial chemistry technique, random peptide libraries prepared by solid phase synthesis or the phage display method, or natural components derived from microorganisms, animals, plants, marine organisms and the like, and the like.

In one mode of embodiment, the screening method of the present invention comprises the following steps (I'), (II') and (III'):
(I') a step for bringing Aβ1-40 into contact with stem cells in the presence of a test substance,
(II') a step for measuring the degree of differentiation into the endothelial precursor in the cells contacted in (I') above, and comparing it with the degree of differentiation in control cells brought into contact with Aβ1-40 in the absence of the test substance,
(III') a step for selecting a test substance that has inhibited the action of Aβ1-40 to suppress endothelial precursor differentiation from stem cells, on the basis of the results of the comparison in (II') above. Culturing conditions, measuring method and the like are the same as those for steps (I), (II) and (III) of the above-described screening method.

The present invention further provides a screening method for a prophylactic/therapeutic substance for Alzheimer's disease, which comprises culturing cells in which vascularization can be induced in the presence and absence of a test substance, and comparing the degree of vascularization in the cells under the two conditions, the substance obtainable by the screening method, and a prophylactic/therapeutic agent for Alzheimer's disease comprising the substance.

The test substance subjected to this screening method may be any commonly known compound or new compound; examples include nucleic acids, saccharides, lipids, proteins, peptides, organic low-molecular compounds, compound libraries prepared using combinatorial chemistry technique, random peptide libraries prepared by solid phase synthesis or the phage display method, or natural components derived from microorganisms, animals, plants, marine organisms and the like, and the like.

In one mode of embodiment, this screening method comprises the following steps (a), (b) and (c):
(a) a step for bringing a test substance and cells in which vascularization can be induced into contact with each other,
(b) a step for measuring the degree of vascularization in the cells contacted in (a) above, and comparing it with the degree of vascularization in control cells not brought into contact with the test substance,
(c) a step for selecting a test substance that promotes vascularization on the basis of the results of the comparison in (b) above.

In step (a) of the above-described method, a test substance is brought into contact with cells in which vascularization can be induced. The contact can be performed in culture medium. The cells can be animal-derived vascular endothelial cells, for examples, vascular endothelial cells, or vascular tissue derived from mammals such as mice, rats, hamsters, guinea pigs, rabbits, dogs, monkeys, and humans and the like.

The culture medium wherein a test substance is brought into contact with cells in which vascularization can be induced is chosen as appropriate according to the kind of cells used and the like; for example, a minimal essential medium (MEM), Dulbecco's modified minimal essential medium (DMEM), RPMI1640 medium, 199 medium and the like, comprising about 5 to 20% fetal bovine serum, can be mentioned. Culturing conditions are also chosen as appropriate according to the kind of cells used and the like; for example, the pH of medium is about 6 to about 8, culturing temperature is normally about 30°C to about 40°C, and culturing time is about 12 hours to about for 72 hours.

In step (b) of the above-described method, first, the degree of promotion of vascularization in the cells contacted with the test substance is measured. The measurement can be performed by a method known per se in consideration of the kind of cells used and the like.

For example, an evaluation can be performed using a kit such as the BD Biocoat TM angiogenesis system, manufactured by Nippon Becton Dickinson Co., Ltd.
(http://www.bdj.co.jp/pdf/35-284-288.pdf)

Next, the degree of promotion of vascularization in the cells contacted with the test substance is compared with the degree of promotion of vascularization in the control cells not contacted with the test substance. The comparison of the degree of promotion of vascularization is preferably performed on the basis of the presence or absence of a significant difference. Although the degree of promotion of vascularization in the control cells not contacted with the test substance may be measured prior to, or simultaneously with, the measurement of the degree of promotion of vascularization in the cells contacted with the test substance, it is preferable from the viewpoint of experimental accuracy and reproducibility that the degree of promotion of vascularization be a simultaneously measured degree of promotion of vascularization.

In step (c) of the above-described method, a test substance that promotes vascularization is selected.

The contact of a test substance and cells in which vascularization can be induced in the screening method of the present invention can also be performed by administering the test substance to an animal. As examples of the animal, non-human mammals such as mice, rats, hamsters, guinea pigs, rabbits, dogs, and monkeys, and the like can be mentioned. In this case, "culturing cells" means that the animal is reared under appropriate rearing conditions.

The screening method of the present invention enables screening for a substance that promotes vascularization. Therefore, the screening method of the present invention is useful for the development of a prophylactic/therapeutic agent for Alzheimer's disease and investigational reagents for the disease, and the like.

An active ingredient of the prophylactic/therapeutic agent of the present invention can be a substance that inhibits the action of Aβ1-40 to suppress Akt/PKB activity and/or a substance that promotes Akt/PKB activity.

As the substance that inhibits Akt/PKB activity suppression and/or the substance that promotes Akt/PKB activity, cytokines such as hepatocyte growth factor (HGF), vascular endothelial growth factor (VEGF), fibroblast growth factor (FGF), epidermal growth factor (EGF), insulin-like growth factor (IGF), nerve growth factor (NGF), platelet-derived growth factor (PDGF), thymidine phosphorylase (TdRPase), platelet-derived endothelial cell growth factor (PD-ECGF), insulin-like growth factor (IGF) and nerve growth factor (NGF) can be mentioned, preferably HGF, VEGF, FGF and EGF, more preferably HGF, VEGF and FGF. These cytokines can be produced by the same method as that for the above-described cytokine that inhibits the action of Aβ1-40 to suppress vascular endothelial precursor cell differentiation and/or cytokine capable of promoting vascularization.

Provided that the substance that inhibits the action of Aβ1-40 to suppress Akt/PKB activity and/or the substance that promotes Akt/PKB activity is a protein or peptide such as the above-described cytokine or a partial peptide thereof, the substance can be formulated in the prophylactic/therapeutic agent of the present invention in the form of a nucleic acid that encodes the same, particularly an expression vector comprising the nucleic acid. In this case as well, the substance can be prepared in the same manner as the above-described nucleic acid that encodes a cytokine that inhibits the action of Aβ1-40 to suppress vascular endothelial precursor cell differentiation and/or a cytokine capable of promoting vascularization, and can be administered to a mammal such as a human in the same manner as a pharmaceutical preparation together with a pharmaceutically acceptable carrier.

The dosage of the prophylactic/therapeutic agent of the present invention varies depending on the activity and kind of active ingredient, seriousness of illness, recipient animal species, the recipient's drug tolerance, body weight, age, and the like, and is normally about 1 µg to about 1000 mg, preferably about 10 µg to about 500 mg, more preferably about 100 µg to about 100 mg, as the amount of active ingredient per day for an adult. This is administered at one time or in several divided portions.

The present invention also provides a screening method for a substance that inhibits the action of Aβ1-40 to suppress protein kinase B activity, which comprises bringing Aβ1-40 into contact with cells capable of expressing protein kinase B activity in the presence and absence of a test substance, and comparing protein kinase B activity under the two conditions, the substance obtainable by the screening method, and a prophylactic/therapeutic agent for Alzheimer's disease comprising the substance.

The test substance subjected to this screening method may be any commonly known compound or new compound; examples include nucleic acids, saccharides, lipids, proteins, peptides, organic low-molecular compounds, compound libraries prepared using combinatorial chemistry technique, random peptide libraries prepared by solid phase synthesis or the phage display method, or natural components derived from microorganisms, animals, plants, marine organisms and the like, and the like.

In one mode of embodiment, the above-described screening method comprises the following steps (i), (ii) and (iii):
(i) a step for bringing Aβ1-40 into contact with cells capable of expressing Akt/PKB activity in the presence of a test substance,
(ii) a step for measuring Akt/PKB activity in the cells contacted in (i) above, and comparing it with Akt/PKB activity in control cells brought into contact with Aβ1-40 in the absence of the test substance,
(iii) a step for selecting a test substance that inhibits the action of Aβ1-40 to suppress Akt/PKB activity on the basis of the results of the comparison in (ii) above.

In step (i) of the above-described method, Aβ1-40 is brought into contact with cells for which Akt/PKB activity can be measured, in the presence of the test substance. The contact can be performed in culture medium. The cells can be animal cells, for example, cells (for example, vascular endothelial cells) derived from mammals such as mice, rats, hamsters, guinea pigs, rabbits, dogs, monkeys, and humans.

The culture medium wherein Aβ1-40 is brought into contact with cells capable of expressing Akt/PKB activity is chosen as appropriate according to the kind of cells used and the like; for example, a minimal essential medium (MEM), Dulbecco's modified Eagle medium (DMEM), RPMI1640 medium, 199 medium and the like, comprising about 5 to 20% fetal bovine serum, can be mentioned. Culturing conditions are also chosen as appropriate according to the kind of cells used and the like; for example, the pH of the medium is about 6 to about 8, culturing temperature is normally about 30°C to about 40°C, and culturing time is about 12 hours to about for 72 hours.

In step (ii) of the above-described method, first, cellular Akt/PKB activity is measured in the cells contacted with Aβ1-40. The measurement can be performed by a method of phosphorylation detection known per se in consideration of the kind of cells used and the like.

For example, the measurement can be performed using detection of auto-phosphorylation of Akt/PKB using an antibody against phosphorylated Akt, mass spectrometry and the like. Alternatively, the activity can be indirectly evaluated using a kit such as the BD Biocoat TM angiogenesis system, manufactured by Nippon Becton Dickinson Co., Ltd.
(http://www.bdj.co.jp/pdf/35-284-288.pdf)

Next, cellular activity in the cells contacted in the presence of the test substance is compared with cell activity in the cells contacted in the absence of the test substance. The comparison of the activity is preferably performed on the basis of the presence or absence of a significant difference. Although the activity in the control cells contacted in the absence of the test substance may be an activity measured prior to, or simultaneously with, the measurement of the activity in the cells contacted in the presence of the test substance, it is preferable from the viewpoint of experimental accuracy and reproducibility that the activity be a simultaneously measured activity.

In step (iii) of the above-described method, a test substance that inhibits the action of Aβ1-40 to suppress Akt/PKB activity is selected.

The screening method of the present invention can also be performed by administering a test substance to an animal. As examples of the animal, non-human mammals such as mice, rats, hamsters, guinea pigs, rabbits, dogs, and monkeys can be mentioned.

The screening method of the present invention enables screening for a substance that inhibits the action of Aβ1-40 to suppress Akt/PKB activity. Therefore, the screening method of the present invention is useful for the development of a prophylactic/therapeutic agent for Alzheimer's disease and investigational reagents for the disease, and the like.

The present invention also provides a screening method for a prophylactic/therapeutic substance for Alzheimer's disease, which comprises culturing cells capable of expressing protein kinase B activity in the presence and absence of a test substance, and comparing protein kinase B activity in the cells under the two conditions. This screening method can be performed in exactly the same manner as the above-described screening method for a substance that inhibits the action of Aβ1-40 to suppress protein kinase B activity, except that Aβ1-40 is not added; a test substance that has increased protein kinase B activity in the presence of the test substance compared to the absence thereof is selected as a candidate for a prophylactic/therapeutic substance for Alzheimer's disease.

### Examples

The present invention is hereinafter described in more detail by means of the following Examples, which, however, are not to be construed as limiting the present invention.

### (Cell culture)

Human brain microvascular endothelial cells (HBMEC), human umbilical artery endothelial cells (HUAEC), human umbilical vein endothelial cells (HUVEC) and human aortic smooth muscle cells (HSMC) were obtained from Applied Cell Biology Research Institute. The cells were cultured using a modified MCDB131 medium supplemented with 2% or 10% FBS; those subcultured over 3 to 6 passages were used in the experiments described below.

### (Example 1)

Human brain microvascular endothelial cells (arterial and venous) cultured in a 12-well plate by the above-described method were stimulated with Aβ1-40 (0.05 µM, 0.5 µM and 5 µM), Aβ1-42 (0.5 µM), Aβ40-1 (0.5 µM), and control (no Aβ derivative added) for 72 hours. Aβ40-1 has the sequence reverse to that of Aβ1-40. This was used as the control peptide. Next, the number of cells was counted manually using a hemocytometer.

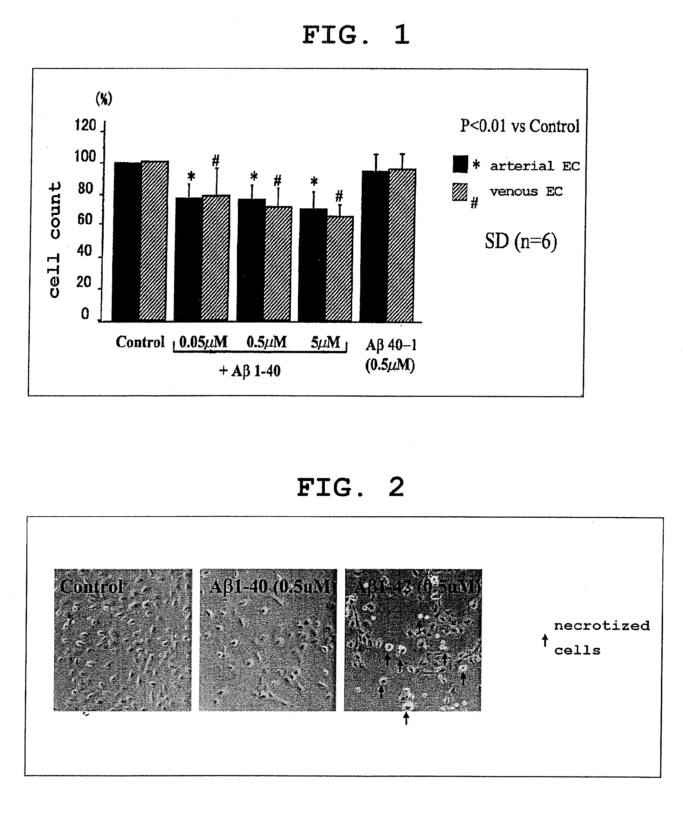

The results of this experiment are shown in Table 1, Figure 1 and Figure 2.

**Table 1**

| Artery endothelial cells | | Venous endothelial cells | |
|---|---|---|---|
| Aβ1-40 (0.05 µm) | 78±9.481 | Aβ1-40 (0.05 µm) | 80±18.987 |
| Aβ1-40 (0.5 µm) | 75±8.011 | Aβ1-40 (0.5 µm) | 73±11.392 |
| Aβ1-40 (5 µm) | 73±11.699 | Aβ1-40 (5 µm) | 70±5.968 |
| Aβ40-1 (0.5 µm) | 97±8.934 | Aβ40-1 (0.5 µm) | 99±6.288 |

| | | | |
|---|---|---|---|
| * Cell count (relative to control as 100) | | | |

It was found that Aβ1-40 suppressed the growth of human brain microvascular endothelial cells, whereas Aβ40-1 did not. This was more conspicuous in arteries. (Figure 1)

Because necrotized cells were observed among the Aβ1-42-stimulated cells, Aβ1-42 was found to have a toxic effect on endothelial cells. (Figure 2)

### (Example 2)

Human umbilical artery endothelial cells and human umbilical vein endothelial cells were stimulated with Aβ1-40 (0.5 µM and 5 µM), Aβ40-1 (0.5 µM), and control (no Aβ derivative added) for 72 hours; after reaching an 80% confluence, endothelial cells were then washed with PBS, trypsinated, and cultured on a 6-well culture plate coated with Matrigel basal membrane matrix (Becton Dickinson Labware) for 18 hours. The cells on the Matrigel were photographed, and the full-length was measured using an NIH image analyzer.

The results are shown in Table 2 and Table 3. The unit of measurement is in pixel.

**Table 2**

| Artery endothelial cells | | | | |
|---|---|---|---|---|
| | Number of rows | Mean value | Standard deviation | Standard error |
| Control | 6 | 11530.333 | 492.232 | 200.953 |
| Aβ1-40 (0.5 µm) | 6 | 8719.333 | 1401.024 | 571.966 |
| Aβ1-40 (5 µm) | 6 | 7951.167 | 634.222 | 258.920 |
| Aβ40-1 (0.5 µm) | 6 | 11863.677 | 894.591 | 365.215 |

**Table 3**

| Vein endothelial cells | | | | |
|---|---|---|---|---|
| | Number of rows | Mean value | Standard deviation | Standard error |
| Control | 6 | 10875.500 | 844.251 | 344.664 |
| Aβ1-40 (0.5 µm) | 6 | 10599.000 | 453.972 | 185.333 |
| Aβ1-40 (5 µm) | 6 | 9884.333 | 978.441 | 399.447 |
| Aβ40-1 (0.5 µm) | 6 | 10875.500 | 1376.358 | 561.896 |

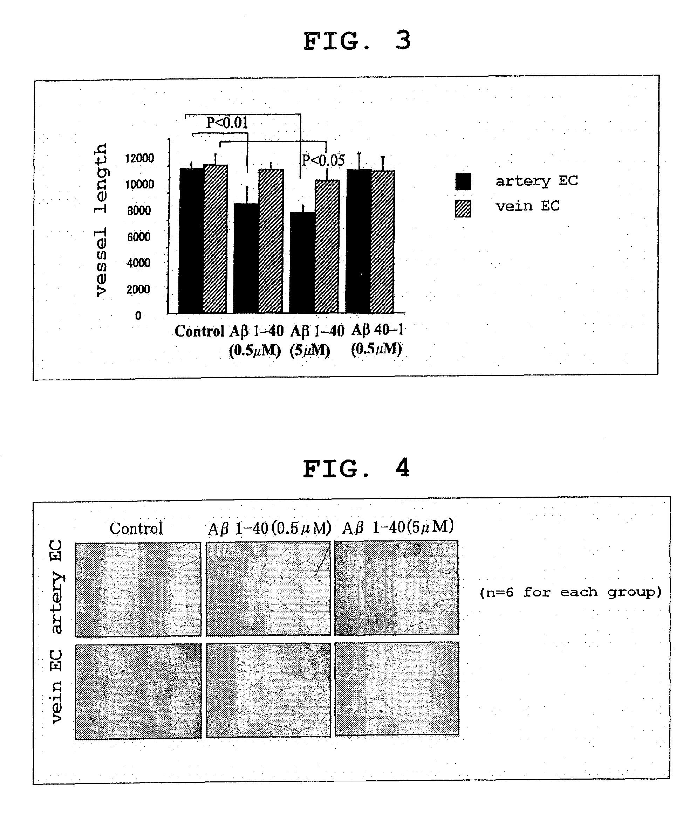

For both Tables 2 and 3, basic statistics: 2 rows, effect: 4 rows. From this result, it was found that Aβ1-40 suppressed EC vessel formation on Matrigel. (Figures 3 and 4)

### (Example 3)

Next, endothelial cell migration was examined using Boyden chamber assay. Briefly, an EBM-2 medium comprising VEGF (10 ng/ml), Aβ1-40 (0.5 µM and 5 µM) +VEGF (10 ng/ml), Aβ40-1 (0.5 µM)+VEGF (10 ng/ml), 10% FBS or control (no Aβ derivative added) was placed in the lower section of a chamber. 5x10⁴ endothelial cells (in 50 µL of EBM-2 supplemented with 0.5% bovine serum albumin) were seeded into the upper section of the chamber. Cell migration was measured by counting the cells in four highly magnified fields of vision (x40). Three experiments were performed for each group. (mean ± standard deviation: P<0.05)

The results are shown in Table 4. The unit of measurement is in cell count/field of vision.

**Table 4**

| | Number of rows | Mean value | Standard deviation | Standard error |
|---|---|---|---|---|
| Control | 4 | 21.080 | 1.225 | 0.612 |
| VEGF | 4 | 100.018 | 42.218 | 20.609 |
| VEGF+Aβ1-40 | 4 | 48.322 | 10.886 | 5.443 |
| VEGF+Aβ40-1 | 4 | 107.257 | 62.944 | 31.472 |
| 10% FBS | 4 | 162.363 | 83.963 | 41.981 |

| | | | | |
|---|---|---|---|---|
| Basic statistics: 3 rows Effect: 1 row | | | | |

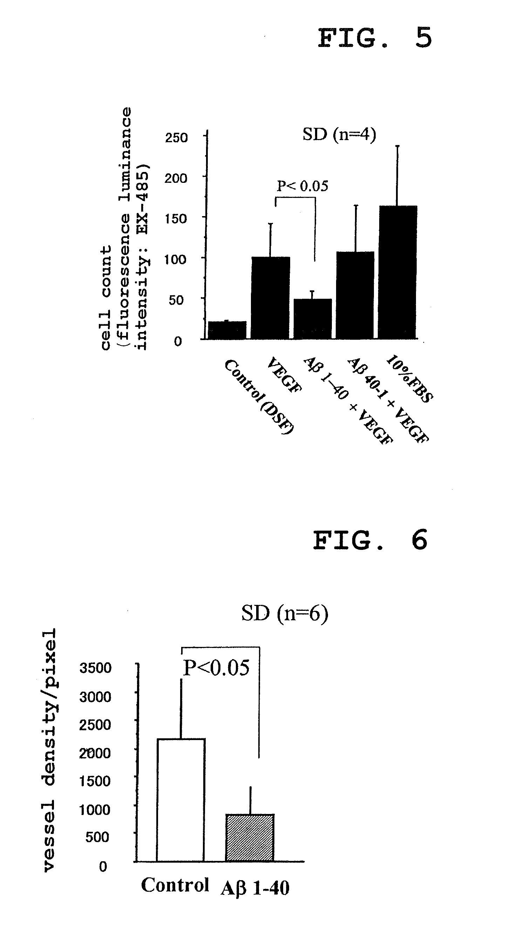

From this, it was found that endothelial migration was caused by vasculogenesis by VEGF, and that the effect was inhibited by Aβ1-40. (Figure 5)

### (Example 4)

### (Matrigel plug transplant assay)

Age-matched male c57/BL6J mice at 8 weeks of age were given 500 µL of Matrigel comprising 50 ng/mL VEGF and Aβ1-40 (5 µM), and 37 units of heparin by subcutaneous injection. Seven days later, the Matrigel plug was carefully recovered from each mouse, washed with phosphate-buffered saline (PBS) three times, and stored under freezing at -20°C. A section (6 mm) of the frozen plug was fixed in ice-cooled acetone, and then stained with rat anti-mouse CD31 primary antibody (Pharmingen), FITC-conjugated anti-α smooth muscle actin antibody (Sigma) and Alexa-conjugated goat anti-rat secondary antibody (Molecular Probe).

Blood vessel density in the stained section was evaluated by fluorescence intensity using the NIH image software. The hemoglobin content in each plug was measured as an index of adult vascular regeneration.

The results are shown in Tables 5 and 6.

**Table 5**

| Blood vessel density (unit of measurement: pixel) | | | | |
|---|---|---|---|---|
| | Number of rows | Mean value | Standard deviation | Standard error |
| Control | 5 | 2171.800 | 1046.239 | 467.892 |
| Aβ1-40 | 5 | 825.800 | 474.909 | 212.386 |

| | | | | |
|---|---|---|---|---|
| Basic statistics: 2 rows Effect: 1 row | | | | |

**Table 6**

| Hemoglobin content (unit of measurement: OD 450/tissue mg) | | | | |
|---|---|---|---|---|
| | Number of rows | Mean value Mean value | Standard deviation | Standard error |
| Control | 5 | 0.251 | 0.079 | 0.035 |
| Aβ1-40 | 5 | 0.084 | 0.040 | 0.018 |

| | | | | |
|---|---|---|---|---|
| Basic statistics: some rows Effect: 4 rows | | | | |

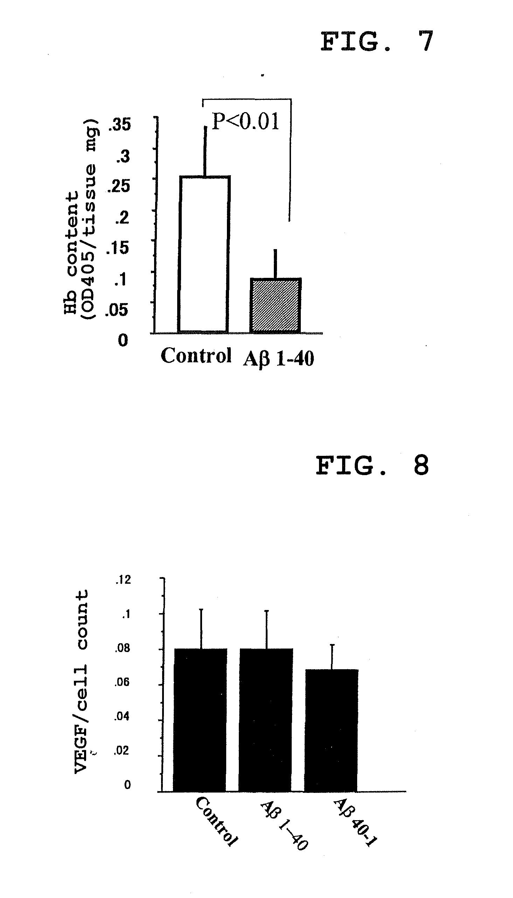

From these results, it was found that Aβ1-40 had a suppressive effect on adult vascular regeneration in vivo. (Figures 6 and 7)

### (Example 5)

### (ELISA assay)

Human SMC was cultured in a 12-well culture plate of an MCDB medium comprising 10% FBS. 48 hours after Aβ1-40 stimulation, the VEGF level in each well was tested by enzyme immunoassay (R&D System).

The results are shown in Table 7.

**Table 7**

| | Number of rows | Mean value | Standard deviation | Standard error |
|---|---|---|---|---|
| Control (DSF)-SMC | 6 | 0.080 | 0.023 | 0.009 |
| Aβ1-40 (DSF) | 6 | 0.080 | 0.022 | 0.009 |
| Aβ40-1 (DSF) | 6 | 0.068 | 0.014 | 0.006 |

From this, it was found that Aβ1-40 had no direct effect on VEGF secretion from VSMC (Figure 8). It was also found that from Dil-acetylated LDL and BS-1, Aβ1-40 had an inhibitory effect on the differentiation of vascular stem cells.

### (Example 6)

### (Western blot assay)

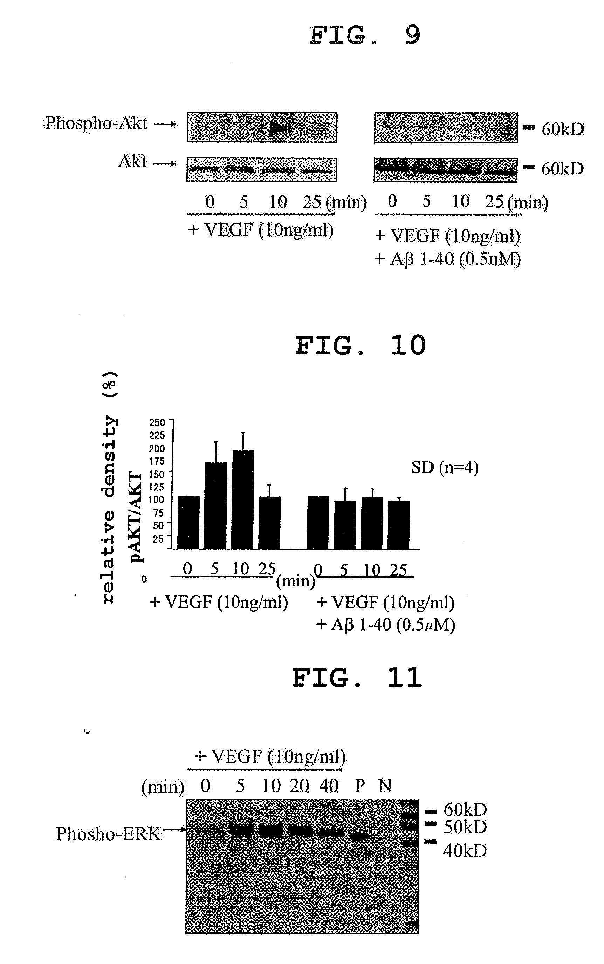

After the EC reached an 80% confluence, the medium was exchanged with an FBS-free fresh medium, and the EC was allowed to stand for 4 hours. Next, the cells were stimulated with human VEGF (10 ng/mL) and Aβ1-40 (0.5 µM) for up to 40 minutes. Total cell protein extract from human endothelial cells was separated on 12.5% SDS-polyacrylamide gel, and transferred to a nitrocellulose membrane. The membrane was subjected to Western blotting using antibodies against human Akt, phosphorylated Akt and phosphorylated ERK (Cell Signaling). The results are shown in Figures 9 to 12.

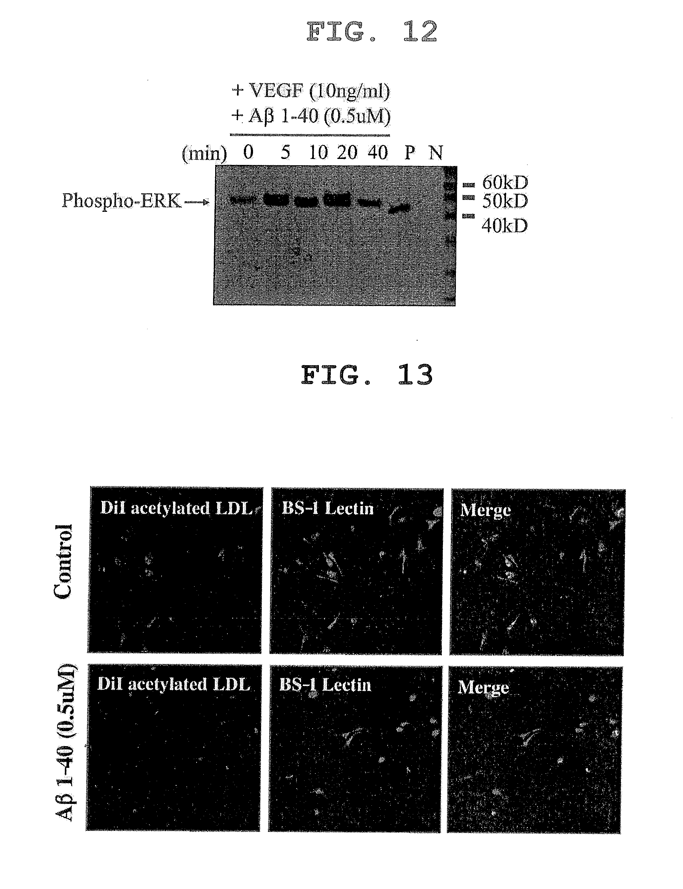

From this, it was found that intracellular Akt phosphorylation was stimulated by VEGF and inhibited by Aβ1-40 (Figure 9). Figure 10 is a graph showing the quantitative inhibition of Akt phosphorylation by Aβ1-40. Aβ1-40 had no effect on the ERK phosphorylation caused by VEGF (Figures 11 and 12).

### (Example 7)

### (Adult vascular stem cell differentiation assay)

Briefly, low-density monocytes were isolated from the spleen of a C57BL/6J mouse by a density centrifugation method using Histopaque-1083 (Sigma, St. Louis, MO). The cells were seeded onto a 4-well glass slide (Nunc International) coated with rat vitronectin (Sigma), and cultured in a medium comprising 2% heat inactivated defined FBS. The medium was supplemented with the endothelial growth supplementation kit, EGM-2MV single aliquot (Clonetics).

After 4 days of cultivation, the cells were incubated with the addition of acetylated LDL (Biomedical Technologies, Stoughton, MA), and stained with FITC-conjugated Griffonia *simplicifolia* lectin I (BS1-lectin) (Vector Laboratories). In 15 fields of vision at 40x magnification, the number of cells double stained with acLDL-Dil and BS1 was counted for each sample as endothelial precursors. The results are shown in Figure 13.

From this, it was found that Aβ1-40 suppressed the growth and differentiation of the cultured vascular precursor cells.

### (Test Example)

### (Aβ-infusion model)

Male ddY mice (6-8 weeks old) were obtained from CLEA Japan. The intracerebroventricular administration was carried out in accordance with the procedure established by Haley and McCormick (Br. J. Pharmacol Chemother. 12(1):12-5, 1957) with minor modifications (Mamiya and Ukai, Br. J. Pharmacol., 134(8):1597-9, 2001). Mice were anesthetized with isoflulane gas, xylazine and ketamine, 5 µl Aβ1-40 (200 µmol/5 µl) was injected i.c.v. into the mice, aimed at 1 mm to 1.5 mm lateral to the midline and 0.5 mm posterior to the bregma by 100 µl-Hamilton syringe with a 3 mm, 27 gauge needle.

Further, a sham operation was performed and sham operated mice (sham group) were prepared.

### (Delivery of HGF gene)

Human HGF cDNA (2.2 kb) was inserted into a pVAX1 plasmid that uses the cytomegalovirus promoter/enhancer. The plasmid was prepared to 10 µg/µl with Milli-Q water. 2 µl plasmid containing human HGF cDNA was suspended in 1.25 µl Optison (Ultrasound contrast medium) and 1 µl TE. 1 week after Aβ1-40 injection into cerebroventricle, pVAX plasmid mixture was injected into cerebroventricle in the same way as injection of Aβ aimed at the opposite side of midline (HGF group). After the infection, the ultrasound (2.0 W/cm², 10 sec.) was applied to each mouse.

Further, pVAX plasmid mixture free of HGF was injected into other mice in the same way to obtain control mice (control group).

### (Test Example 1)

### (Water finding Task)

1 week after the administration of plasmid containing human HGF cDNA, the water finding task was carried out according to the method of Nabeshima et al. (Brain Res., 848(1-2):167-73, 1999). Briefly, the apparatus consisted of an open field (50 X 30 cm with walls 15 cm high) with an alcove (10 cm X 10 cm X 10 cm) at the middle of one of the longer sides of the wall. The floor of the open field was gridded every 10 cm for measuring locomotor activity. A water bottle was inserted into the ceiling of the alcove with its tube tip 5 cm in the training trial or 7 cm in the retention trial above the floor. The test consisted of two trials: a training trial (Day 1) and a retention trial (Day 2). In the training trial, mice were placed individually into one corner of the opposite side of alcove and allowed to explore freely for 3 min. During this time, ambulation was measured by counting the number of grid lines that the mice crossed. The frequency of touching, sniffing, or licking of the water tube in the alcove (the umber of approaches) was also counted. The mice that could not find the water tube within 3 min were excluded from the test. The mice were returned immediately to their home cages after the training trial, and mice were then deprived of water for 48 hours to be motivated to look for water. In the retention trial, the mice were placed individually in the same corner of the apparatus. To evaluate memory acquisition, drinking latency (DL) was measured as the time from leaving the corner to their first touch on the water tube. In addition, the time from the placement to the arrival at the starting corner (Starting Latency SL), and the time from the starting corner to their first entry into the alcove (Entering Latency EL) were measured.

### (Test Example 2)

### (Y-Maze test)

1 week after the administration of plasmid containing human HGF cDNA, the Y-Maze test was carried out in accordance with the aforementioned procedure of Mamiya and Ukai (ibid., 2001). Briefly, the Y-Maze consisted of three arms (a, b, c). Each arm is 40 cm long, whose cross section has trapezoidal shape, with a top of 10 cm, a bottom of 3 cm, and a height of 12 cm (Nabeshima et al, ibid., 1999)). The Y-Maze apparatus was set up on the floor of the experimental room and lit at 200 lux. Each mouse was placed at the end of one arm and allowed to explore the Y-Maze freely for 8 min, and the series of arm entries were recorded to evaluate short-term/spatial working memory by spontaneous alternation behavior. Mice were considered to have chosen an arm when their entire torsos entered the arm. Spontaneous alternation behavior is based upon the behavior of animals preferring choosing newer locations. When mice chose a third arm different from the previous two in the consecutive three arm choices in the Y-Maze, the mice were considered to have carried out actual alternation. Alternation behavior (%) was calculated as the percentage of actual alternations to maximum alternations that were defined as the total number of arm entries minus two. For example, if a mouse made 10 entries in the order of Arm a-b-c-b-a-b-c-a-b-c, the actual and maximum alternations would be 6 and 8, respectively, and alternation behavior would be 75%. The number of arm entries, defecations, and urinations were counted.

### (Results)

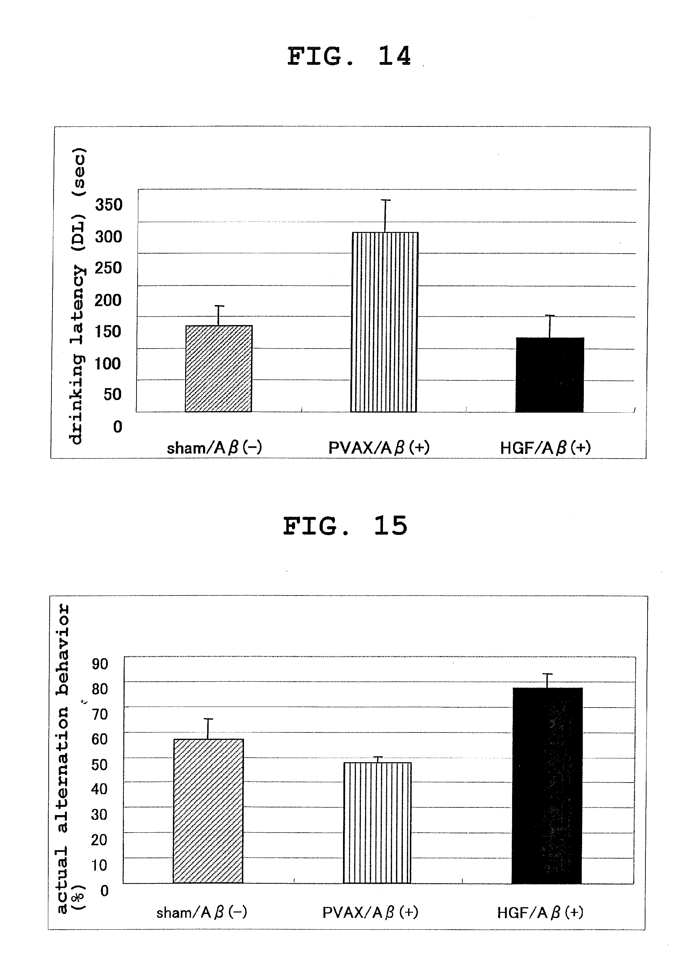

The results of Test Example 1 are shown in Table 8 and Figure 14; the results of Test Example 2 are shown in Table 9 and Figure 15. The Aβ-injected model mice transfected with the HGF gene exhibited improved behavior in the water finding test and Y-Maze test.

**Table 8**

| Test Example 1. Water finding test | | | | | | |
|---|---|---|---|---|---|---|
| | SL (sec) | Mean Standard deviation | EL (sec) | Mean Standard deviation | DL (sec) | Mean Standard deviation |
| sham | 8 | | 38 | | 64 | |
| sham | 8 | 9.00 0.58 | 154 | 82.50 28.77 | 210 | 135.75 31.32 |
| sham | 10 | | 85 | | 158 | |
| sham | 10 | | 53 | | 111 | |
| pVAX | 4 | | 55 | | 340 | |
| pVAX | 175 | | 280 | | 384 | |
| pVAX | 21 | 40.00 23.37 | 32 | 112.43 35.82 | 399 | 283.00 50.23 |
| pVAX | 53 | | 191 | | 238 | |
| pVAX | 10 | | 80 | | 391 | |
| pVAX | 7 | | 20 | | 49 | |
| pVAX | 10 | | 129 | | 180 | |
| HGF | 5 | | 14 | | 86 | |
| HGF | 11 | 8.75 1.65 | 41 | 25.75 5.84 | 114 | 116.50 36.05 |
| HGF | 12 | | 28 | | 217 | |
| HGF | 7 | | 20 | | 49 | |

**Table 9**

| Test Example 2. Y-Maze test | | | |
|---|---|---|---|
| | Detail of alternation behavior Total alternations | Alternation behavior (%) | Mean Standard deviation |
| sham | bcbababacababacbcababa | 35.0 | |
| sham | abacabacbabaca | 50.0 | 56.88 8.09 |
| sham | cbacbacbacbacbabacbacabcabc | 84.0 | |
| sham | cabcacbacabacbababacabacba | 62.5 | |
| sham | acbacabacacbcabacac | 52.9 | |
| pVAX | caacabcbabca | 50.0 | |
| pVAX | bcacacabacbacabacbaa | 57.9 | |
| pVAX | cbacaccbacabacbabcacacabacbabababcbac | 48.6 | 47.94 2.35 |
| pVAX | cbcbabaabcabacacbacbcacacacbc | 44.0 | |
| pVAX | bababacacbabacacbcabacaba | 39.1 | |
| pVAX | acabcabacacabaccca | 43.8 | |
| pVAX | bcabcabcabacabcbcbabababa | 52.2 | |
| HGF | cbacbcbcacbcbcbacbacbcbabcabc | 59.3 | |
| HGF | cbacbacacbcabacbcacbacbcacbacbacabc | 72.7 | 77.70 5.71 |
| HGF | abcabcabcabcababcabcabcabcab | 92.3 | |
| HGF | bcabacbacbacbacbcbacbacb | 86.4 | |
| HGF | abcbcbcabcabcabcabca | 77.8 | |

In the water finding test (see Figure 14), the HGF-treated Aβ-injected mice exhibited significantly better behavior than the control mice (p<0.05). There was no significant difference between the sham-operated mice and control mice. However, the sham-operated mice tended to behave better than the control mice (p=0.07).

In the Y-Maze test as well (see Figure 15), the HGF-treated Aβ-injected mice exhibited significantly better behavior than the control mice (p<0.05). There was no significant difference between the sham-operated mice and control mice. However, the sham-operated mice tended to behave better than the control mice (p=0.24).

### Industrial Applicability

The prophylactic/therapeutic agent provided by the present invention is useful for Alzheimer's disease. Alternatively, the prophylactic/therapeutic agent of the present invention is useful as an investigational reagent for Alzheimer's disease. The screening method provided by the present invention is useful for the development of a prophylactic/therapeutic drug for Alzheimer's disease or investigational reagents for the disease.

This application is based on a patent application No. 2004-347861 filed in Japan, the contents of which are incorporated in full herein by this reference.

## Claims

1. A prophylactic/therapeutic agent for Alzheimer's disease comprising a substance that inhibits the action of β-amyloid 1-40 to suppress vascular endothelial precursor cell differentiation and/or endothelial precursor differentiation from stem cells and/or a substance that promotes vascularization as an active ingredient.

2. The agent of claim 1, wherein the substance that inhibits the action of β-amyloid 1-40 to suppress vascular endothelial precursor cell differentiation and/or endothelial precursor differentiation from stem cells and/or the substance that promotes vascularization is a cytokine.

3. The agent of claim 2, wherein the cytokine that inhibits the action of β-amyloid 1-40 to suppress vascular endothelial precursor cell differentiation and/or endothelial precursor differentiation from stem cells is one or more selected from the group consisting of hepatocyte growth factor (HGF), vascular endothelial growth factor (VEGF), fibroblast growth factor (FGF), epidermal growth factor (EGF), insulin-like growth factor (IGF) and nerve growth factor (NGF).

4. The agent of claim 2, wherein the cytokine that promotes vascularization is one or more selected from the group consisting of hepatocyte growth factor (HGF), vascular endothelial growth factor (VEGF), fibroblast growth factor (FGF), epidermal growth factor (EGF), platelet-derived growth factor (PDGF), thymidine phosphorylase (TdRPase), platelet-derived endothelial cell growth factor (PD-ECGF), insulin-like growth factor (IGF) and nerve growth factor (NGF).

5. A prophylactic/therapeutic agent for Alzheimer's disease comprising a nucleic acid that encodes a protein that inhibits the action of β-amyloid 1-40 to suppress vascular endothelial precursor cell differentiation and/or endothelial precursor differentiation from stem cells and/or a protein that promotes vascularization.

6. A screening method for a substance that inhibits the action of β-amyloid 1-40 to suppress vascular endothelial precursor cell differentiation, which comprises bringing β-amyloid 1-40 into contact with vascular endothelial precursor cells in the presence and absence of a test substance, and comparing the degree of differentiation of the cells under the two conditions.

7. A screening method for a substance that inhibits the action of β-amyloid 1-40 to suppress endothelial precursor differentiation from stem cells, which comprises bringing β-amyloid 1-40 into contact with stem cells in the presence and absence of a test substance, and comparing the degree of differentiation of the cells into the endothelial precursor under the two conditions.

8. The method of claim 6 or 7, which is a screening method for a prophylactic/therapeutic substance for Alzheimer's disease.

9. A screening method for a prophylactic/therapeutic substance for Alzheimer's disease, which comprises culturing cells in which vascularization can be induced in the presence and absence of a test substance, and comparing the degree of vascularization in the cells under the two conditions.

10. A prophylactic/therapeutic agent for Alzheimer's disease comprising a substance that inhibits the action of β-amyloid 1-40 to suppress protein kinase B activity and/or a substance that promotes protein kinase B activity as an active ingredient.

11. The agent of claim 10, wherein the substance that inhibits the action of β-amyloid 1-40 to suppress protein kinase B activity and/or the substance that promotes protein kinase B activity is a cytokine.

12. The agent of claim 11, wherein the cytokine is one or more selected from the group consisting of hepatocyte growth factor (HGF), vascular endothelial growth factor (VEGF), fibroblast growth factor (FGF), epidermal growth factor (EGF), insulin-like growth factor (IGF), nerve growth factor (NGF), platelet-derived growth factor (PDGF), thymidine phosphorylase (TdRPase), platelet-derived endothelial cell growth factor (PD-ECGF), insulin-like growth factor (IGF) and nerve growth factor (NGF).

13. A prophylactic/therapeutic agent for Alzheimer's disease comprising a nucleic acid that encodes a protein that inhibits the action of β-amyloid 1-40 to suppress protein kinase B activity and/or a substance that promotes protein kinase B activity.

14. A screening method for a substance that inhibits the action of β-amyloid 1-40 to suppress protein kinase B activity, which comprises bringing β-amyloid 1-40 into contact with cells capable of expressing protein kinase B activity in the presence and absence of a test substance, and comparing protein kinase B activity under the two conditions.

15. The method of claim 14, which is a screening method for a prophylactic/therapeutic substance for Alzheimer's disease.

16. A screening method for a prophylactic/therapeutic substance for Alzheimer's disease, which comprises culturing cells capable of expressing protein kinase B activity in the presence and absence of a test substance, and comparing protein kinase B activity in the cells under the two conditions.

17. A prophylactic/therapeutic method for Alzheimer's disease in an animal, which comprises administering an effective amount of a substance that inhibits the action of β-amyloid 1-40 to suppress vascular endothelial precursor cell differentiation and/or endothelial precursor differentiation from stem cells and/or a substance that promotes vascularization to the animal.

18. A prophylactic/therapeutic method for Alzheimer's disease in an animal, which comprises administering an effective amount of a substance that inhibits the action of β-amyloid 1-40 to suppress protein kinase B activity and/or a substance that promotes protein kinase B activity to the animal.

19. A use of a substance that inhibits the action of β-amyloid 1-40 to suppress vascular endothelial precursor cell differentiation and/or endothelial precursor differentiation from stem cells and/or a substance that promotes vascularization, for manufacturing a prophylactic/therapeutic agent for Alzheimer's disease.

20. A use of a substance that inhibits the action of β-amyloid 1-40 to suppress protein kinase B activity and/or a substance that promotes protein kinase B activity, for manufacturing a prophylactic/therapeutic agent for Alzheimer's disease.
